Europäisches Patentamt

European Patent Office

Office européen des brevets

(11) Veröffentlichungsnummer: **0 470 445 A1**

# EUROPÄISCHE PATENTANMELDUNG

(21) Anmeldenummer: **91112478.2**

(51) Int. Cl.5: **C07D 213/803**

(22) Anmeldetag: **25.07.91**

(30) Priorität: **08.08.90 DE 4025076**

(43) Veröffentlichungstag der Anmeldung:
**12.02.92 Patentblatt 92/07**

(84) Benannte Vertragsstaaten:
**AT BE CH DE DK ES FR GB IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Weber, Jürgen, Dr. Dipl.-Chem.**
**Bunsenstrasse 17**
**W-4200 Oberhausen 11(DE)**
Erfinder: **Lappe, Peter, Dr. Dipl.-Chem.**
**Eickenhof 34**
**W-4220 Dinslaken(DE)**
Erfinder: **Springer, Helmut, Dipl.-Ing.**
**Borbecker Strasse 19**
**W-4200 Oberhausen 11(DE)**

(54) **Verfahren zur Herstellung von Estern der 5-Alkylpyridin-2,3-dicarbonsäure.**

(57) 5-Alkylpyridin-2,3-dicarbonsäureester werden durch Umsetzung von 2-Oxobernsteinsäureestern mit 2-Alkylacroleinoximen erhalten.

EP 0 470 445 A1

Die Erfindung betrifft ein Verfahren zur Herstellung von Estern der 5-Alkylpyridin-2,3-dicarbonsäure durch Umsetzung von 2-Alkylacroleinoximen mit 2-Oxobernsteinsäureestern.

Derivate der Pyridin-2,3-dicarbonsäure, vor allem die 5-Alkylpyridin-2,3-dicarbonsäuren, sind wertvolle Zwischenprodukte für die Herstellung von 2-(2-Imidazolin-2-yl)-nicotinsäure-Verbindungen, die ausgeprägte herbizide Wirkung besitzen. Insbesondere die (R,S)-5-Ethyl-2-(4-isopropyl-4-methyl-5-oxo-2-imidazolin-2-yl)-nicotinsäure ist von wirtschaftlichem Interesse. Die Herstellung dieser Verbindung ist beispielsweise Gegenstand der US-PS 4 460 776.

Entsprechend ihrer Bedeutung sind für die Herstellung von Pyridin-2,3-dicarbonsäurederivaten eine Reihe Synthesen bekannt. Nach der Lehre der EP 0 274 379 A2 erhält man Verbindungen der Pyridin-2,3-dicarbonsäure entweder durch Umsetzung von 3-Halogen-2-ketobernsteinsäureestern mit $\alpha,\beta$-ungesättigten Aldehyden oder Ketonen und Ammoniak oder aus Aminobutendisäureestern und $\alpha,\beta$-ungesättigten Aldehyden oder Ketonen. Diese Verfahren gehen entweder von halogenhaltigen Verbindungen aus, die den Einsatz halogenresistenter Apparaturen erfordern und zu umweltbelastenden Halogenverbindungen als Abfallprodukten führen, oder von technisch nicht oder nicht ausreichend verfügbaren Substanzen. Sie sind daher nur begrenzt anwendbar.

Die in der EP 0 299 362 Al beschriebene Synthese von Pyridin-2,3-dicarbonsäure-derivaten beruht ebenfalls auf dem Einatz von Aminobutendisäureestern und $\alpha,\beta$-ungesättigten Carbonylverbindungen. Beide Ausgangsstoffe müssen in reiner Form eingesetzt werden. Der mit dieser Forderung verbundene hohe Destillationsaufwand stellt die Wirtschaftlichkeit des Verfahrens in Frage.

Das Verfahren der EP 0 308 084 Al zur Herstellung von Pyridinderivaten geht von Halogenpropensäurederivaten oder von Halogenbutensäurederivaten aus, die mit einem 2-Alkylacroleinoxim umgesetzt werden. Ein Beispiel für diesen Syntheseweg ist die Herstellung der 5-Ethyl-2,3-pyridindicarbonsäure aus Chlor-N-phenylmaleinimid und 2-Ethylacroleinoxim. Abgesehen davon, daß Chlor-N-phenylmaleinimid nur durch eine aufwendige Folge mehrerer Reaktionsschritte aus Maleinsäureanhydrid erhalten werden kann, erfordert das Verfahren lange Reaktionszeiten, die bis zu 60 h betragen und liefert die gewünschte Verbindung nur in mäßigen Ausbeuten.

Wie ersichtlich, erfüllen die bekannten Verfahren nicht alle technischen und wirtschaftlichen Voraussetzungen, denen industriell ausgeübte Prozesse genügen müssen. Teils beruhen sie auf dem Einsatz von Ausgangsstoffen, deren Herstellung aufwendig ist oder die bei der Synthese oder der Weiterverarbeitung zu umweltbelastenden Nebenprodukten führen, teils erfordern sie lange Reaktionszeiten oder liefern die erwünschten Verbindungen nur in unzureichender Ausbeute.

Die vorliegende Erfindung hat es sich zum Ziel gesetzt, die Nachteile des Standes der Technik zu beheben und ein Verfahren zur Herstellung von Estern der 5-Alkylpyridin-2,3-dicarbonsäure bereitzustellen, das den Forderungen der technischen Praxis genügt.

Die Erfindung besteht in einem Verfahren zur Herstellung von Estern der 5-Alkylpyridin-2,3-dicarbonsäure. Es ist dadurch gekennzeichnet, daß man 2-Oxobernsteinsäureester in Gegenwart saurer Katalysatoren mit 2-Alkylacroleinoximen umsetzt.

Die Ausgangsstoffe für das erfindungsgemäße Verfahren sind handelsübliche oder aus handelsüblichen Grundchemikalien nach bekannten Arbeitsmethoden leicht zugängliche Substanzen.

Unter den Oxobernsteinsäureestern (Oxalessigsäureester) ist der Oxobernsteinsäurediethylester die bekannteste Verbindung. Sie wird aus Oxalsäurediethylester und Essigsäureethylester durch Claisen-Kondensation in Gegenwart von Natriumalkoholat synthetisiert. Andere Ester, auch gemischte, sind auf demselben Wege aus den entsprechenden Estern der Oxalsäure und der Essigsäure zugänglich. Nach dem erfindungsgemäßen Verfahren kann man die freien Diester einsetzen. Mit gleich gutem Erfolg ist es aber auch möglich, vom Alkalisalz der Enolform des Diesters auszugehen, das durch Umsetzung mit der äquivalenten Menge oder einem geringen Überschuß einer Säure, zweckmäßig einer Mineralsäure in das Enol überführt wird.

Die 2-Alkylacroleinoxime werden in bekannter Weise aus den Aldehyden und Hydroxylaminsalzen erhalten. Zu den 2-Alkylacroleinen gelangt man durch Aldoladdition von gesättigten, aliphatischen Aldehyden an Formaldehyd z.B. in Gegenwart eines Katalysatorsystems aus sekundären Aminen und einer aliphatischen Carbonsäure. 2-Ethylacrolein enthält man z.B. auf diesem Wege aus n-Butyraldehyd und Formaldehyd. Gemäß der Erfindung setzt man Alkylacroleinoxime ein, in denen der Alkylrest ein bis zehn Kohlenstoffatome enthält und geradkettig oder verzweigt ist.

Das erfindungsgemäße Verfahren kann in einfacher Weise durch Mischen der beiden Reaktanten in Gegenwart eines sauren Katalysators, gegebenenfalls bei erhöhter Temperatur durchgeführt werden. Zweckmäßig, jedoch nicht zwingend erforderlich ist es, die Umsetzung in einem inerten Lösungsmittel vorzunehmen. Diese Reaktionsvariante wird vor allem immer dann angewendet, wenn man nicht von freien Oxobernsteinsäurediestern ausgeht, sondern von deren Alkalienolat. Dann setzt man die wäßrige Lösung der Alkaliverbindung mit der Säure um und extrahiert den Diester mit dem inerten Lösungsmittel. Die so erhaltene Lösung wird darauf mit dem 2-Alkylacroleinoxim umgesetzt. Als Lösungsmittel für die Reaktionen eignen sich Alkohole wie Butanol, Ethylenglykol, Ester, z.B. Ethylacetat, Halogenkohlenwasserstoffe, beispielsweise Chloroform oder Tetrachlorkohlenstoff, aromatische Kohlenwasserstoffe wie Benzol, Toluol oder Xylol. Zweckmäßig beträgt die Diester-Konzentration in der Lösung 10 bis 50 Gew.-%, bezogen auf die Lösung.

Die Zugabe eines Katalysators zu dem Reaktionsgemisch verkürzt die Reaktionszeit und unterdrückt unerwünschte Nebenreaktionen. Als Katalysatoren dienen sauer reagierende Verbindungen wie starke, anorganische Säuren, z.B. Schwefelsäure, Salzsäure, organische Sulfonsäuren, z.B. Methansulfonsäure, p-Toluolsulfonsäure, organische Carbonsäuren, z.B. Essigsäure. Bevorzugt werden organische Sulfonsäuren. Die Katalysatormenge beträgt 0,01 bis 0,2, vorzugsweise 0,05 bis 0,1 mol Säure je mol Diester.

Zu der den Katalysator enthaltenden Diesterlösung wird 2-Alkylacroleinoxim, unverdünnt oder in demselben Lösungsmittel gelöst wie der Diester, anteilsweise zugesetzt. Die Reaktanten können im Molverhältnis 1 : 1 eingesetzt werden. Es empfiehlt sich jedoch, einen der beiden Reaktanten im Überschuß zu verwenden, bewährt hat sich ein Molverhältnis Diester zu Oxim von 1 : 2 bis 2 : 1.

Die Umsetzung der Ausgangsstoffe erfolgt bei erhöhter Temperatur. Sie hängt von dem gewählten Lösungsmittel und dem Katalysator ab. Üblicherweise wählt man einen Temperaturbereich zwischen 50 und 150 °C, vorzugsweise 80 bis 100 °C. Höhere Temperaturen sollte man wegen der thermischen Instabilität der Oxime vermeiden. Nach Beendigung der Umsetzung, sie erfordert je nach Reaktionstemperatur 4 bis 10 Stunden, wird das 5-Alkylpyridin-2,3-dicarbonsäurederivat aus dem Reaktionsgemisch unter Anwendung üblicher Arbeitsmethoden isoliert und gereinigt. Geeignete Arbeitsmethoden sind die Extraktion mit einem Lösungsmittel, die Destillation, die Rekristallisation und die Chromatographie.

Nach der Erfindung werden 5-Alkylpyridin-2,3-dicarbonsäureester in hoher Ausbeute und hoher Reinheit in kurzer Reaktionszeit aus billigen und leicht zugänglichen Ausgangsstoffen erhalten.

Die Erfindung wird durch die nachfolgenden Beispiele näher erläutert, jedoch nicht auf die beschriebenen Ausführungsformen beschränkt.

**Beispiel 1**

In einem 2 l-Rundkolben werden 740 g Toluol, 250 g Wasser und 117 g Salzsäure (32 %ig) vorgelegt und innerhalb von 5 Minuten bei 25 °C mit 209,5 g 2-Oxobernsteinsäurediethylester-Na-Salz versetzt. Nach einer Reaktionszeit von 1 Stunde bei 25 °C erfolgt eine Phasentrennung.

Die organische Phase (924,3 g) wird nach Zugabe von 9,36 g p-Toluolsulfonsäure auf 65 °C erwärmt, bei dieser Temperatur werden innerhalb von 15 Minuten 141,8 g 2-Ethylacroleinoxim (90,8 %ig) zugetropft. Nach einer Reaktionszeit von 7 Stunden bei 84 bis 90 °C läßt man auf Raumtemperatur abkühlen, trennt die organische Phase (1056,1 g) von der Wasserphase und wäscht dreimal mit je 300 g Wasser.

Die verbleibende organische Phase (1021,2 g) wird im Vakuum einer Wasserstrahlpumpe bei einer Badtemperatur von 95 °C vom Lösungsmittel befreit. Der Rückstand (261,1 g) enthält nach GLC-Analyse 43,9 % 5-Ethylpyridin-2,3-dicarbonsäurediethylester, dies entspricht einer Ausbeute von 45,7 % der Theorie, bezogen auf eingesetztes 2-Oxobernsteinsäurediethylester-Na-Salz.

**Beispiel 2**

In einem 2 l-Rundkolben werden 740 g Xylol, 250 g Wasser und 117 g Salzsäure (32 %ig) vorgelegt und innerhalb von 5 Minuten bei 25 °C mit 209,5 g 2-Oxobernsteinsäurediethylester-Na-Salz versetzt. Nach einer Reaktionszeit von 1 Stunde bei 25 °C erfolgt eine Phasentrennung.

Die organische Phase (922,7 g) wird nach Zugabe von 9,36 g p-Toluolsulfonsäure auf 65 °C erwärmt, bei dieser Temperatur werden innerhalb von 15 Minuten 141,8 g 2-Ethylacroleinoxim (90,8 %ig) zugetropft. Nach einer Reaktionszeit von 7 Stunden bei 90 bis 97 °C läßt man auf Raumtemperatur abkühlen, trennt die organische Phase (1058,7 g) von der Wasserphase und wäscht dreimal mit je 300 g Wasser.

Die verbleibende organische Phase (1017,6 g) wird im Vakuum einer Wasserstrahlpumpe bei einer Badtemperatur von 95 °C vom Lösungsmittel befreit. Der Rückstand (233,4 g) enthält nach GLC-Analyse 45,5 % 5-Ethylpyridin-2,3-dicarbonsäurediethylester, dies entspricht einer Ausbeute von 42,3 % der Theorie, bezogen auf eingesetztes 2-Oxobernsteinsäurediethylester-Na-Salz.

**Beispiel 3**

In einem 2 l-Rundkolben werden 740 g Toluol, 250 g Wasser und 117 g Salzsäure (32 %ig) vorgelegt und innerhalb von 5 Minuten bei 25 °C mit 209,5 g 2-Oxobernsteinsäurediethylester-Na-Salz versetzt. Nach einer Reaktionszeit von 1 Stunde bei 25 °C erfolgt eine Phasentrennung.

Die organische Phase (925,2 g) wird nach Zugabe von 18,7 g p-Toluolsulfonsäure auf 65 °C erwärmt, bei dieser Temperatur werden innerhalb von 15 Minuten 141,8 g 2-Ethylacroleinoxim (90,8 %ig) zugetropft. Nach einer Reaktionszeit von 7 Stunden bei 86 bis 91°C läßt man auf Raumtemperatur abkühlen, trennt die organische Phase (1055,4 g) von der Wasserphase und wäscht dreimal mit je 300 g Wasser.

Die verbleibende organische Phase (1014,2 g) wird im Vakuum einer Wasserstrahlpumpe bei einer Badtemperatur von 95°C vom Lösungsmittel befreit. Der Rückstand (245,7 g) enthält nach GLC-Analyse 53,2 % 5-Ethylpyridin-2,3-dicarbonsäurediethylester, dies entspricht einer Ausbeute von 52,1 % der Theorie, bezogen auf eingesetztes 2-Oxobernsteinsäurediethylester-Na-Salz.

Beispiel 4

In einem 2 1-Rundkolben werden 740 g Toluol, 250 g Wasser und 117 g Salzsäure (32 %ig) vorgelegt und innerhalb von 5 Minuten bei 25°C mit 209,5 g 2-Oxobernsteinsäurediethylester-Na-Salz versetzt. Nach einer Reaktionszeit von 1 Stunde bei 25°C erfolgt eine Phasentrennung.

Die organische Phase (924,5 g) wird nach Zugabe von 9,4 g p-Toluolsulfonsäure auf 65°C erwärmt, bei dieser Temperatur werden innerhalb von 15 Minuten 130,9 g 2-Ethylacroleinoxim (90,8 %ig) zugetropft. Nach einer Reaktionszeit von 7 Stunden bei 84 bis 89°C läßt man auf Raumtemperatur abkühlen, trennt die organische Phase (1053,2 g) von der Wasserphase und wäscht dreimal mit je 300 g Wasser.

Die verbleibende organische Phase (1018,6 g) wird im Vakuum einer Wasserstrahlpumpe bei einer Badtemperatur von 95°C vom Lösungsmittel befreit. Der Rückstand (242,2 g) enthält nach GLC-Analyse 44,1 % 5-Ethylpyridin-2,3-dicarbonsäurediethylester, dies entspricht einer Ausbeute von 42,6 % der Theorie, bezogen auf eingesetztes 2-Oxobernsteinsäurediethylester-Na-Salz.

Beispiel 5

In einem 2 1-Rundkolben werden 740 g Toluol, 250 g Wasser und 117 g Salzsäure (32 %ig) vorgelegt und innerhalb von 5 Minuten bei 25°C mit 209,5 g 2-Oxobernsteinsäurediethylester-Na-Salz versetzt. Nach einer Reaktionszeit von 1 Stunde bei 25°C erfolgt eine Phasentrennung.

Die organische Phase (922,3 g) wird nach Zugabe von 9,4 g p-Toluolsulfonsäure auf 30°C erwärmt, bei dieser Temperatur werden innerhalb von 15 Minuten 141,8 g 2-Ethylacroleinoxim (90,8 %ig) zugetropft. Nach einer Reaktionszeit von 7 Stunden bei 83 bis 90°C läßt man auf Raumtemperatur abkühlen, trennt die organische Phase (1059,2 g) von der Wasserphase und wäscht dreimal mit je 300 g Wasser.

Die verbleibende organische Phase (1024,0 g) wird im Vakuum einer Wasserstrahlpumpe bei einer Badtemperatur von 95°C vom Lösungsmittel befreit. Der Rückstand (253,3 g) enthält nach GLC-Analyse 47,2 % 5-Ethylpyridin-2,3-dicarbonsäurediethylester, dies entspricht einer Ausbeute von 47,6 % der Theorie, bezogen auf eingesetztes 2-Oxobernsteinsäurediethylester-Na-Salz.

Beispiel 6

In einem 2 1-Rundkolben werden 740 g Toluol, 250 g Wasser und 117 g Salzsäure (32 %ig) vorgelegt und innerhalb von 5 Minuten bei 25°C mit 209,5 g 2-Oxobernsteinsäurediethylester-Na-Salz versetzt. Nach einer Reaktionszeit von 1 Stunde bei 25°C erfolgt eine Phasentrennung.

Die organische Phase (922,8 g) wird nach Zugabe von 9,4 g p-Toluolsulfonsäure auf 85°C erwärmt, bei dieser Temperatur werden innerhalb von 15 Minuten 141,8 g 2-Ethylacroleinoxim (90,8 %ig) zugetropft. Nach einer Reaktionszeit von 7 Stunden bei 84 bis 90°C läßt man auf Raumtemperatur abkühlen, trennt die organische Phase (1062,1 g) von der Wasserphase und wäscht dreimal mit je 300 g Wasser.

Die verbleibende organische Phase (1020,6 g) wird im Vakuum einer Wasserstrahlpumpe bei einer Badtemperatur von 95°C vom Lösungsmittel befreit. Der Rückstand (249,6 g) enthält nach GLC-Analyse 44,4 % 5-Ethylpyridin-2,3-dicarbonsäurediethylester, dies entspricht einer Ausbeute von 44,2 % der Theorie, bezogen auf eingesetztes 2-Oxobernsteinsäurediethylester-Na-Salz.

**Patentansprüche**

1. Verfahren zur Herstellung von Estern der 5-Alkylpyridin-2,3-dicarbonsäure, dadurch gekennzeichnet, daß man 2-Oxobernsteinsäureester in Gegenwart saurer Katalysatoren mit 2-Alkylacroleinoximen umsetzt.

2. Verfahren nach Anspruch 1, dadurch gekennzeichnet, daß die Umsetzung des 2-Oxobernsteinsäureesters mit dem 2-Alkylacroleinoxim in Gegenwart eines Lösungsmittels erfolgt.

3. Verfahren nach Anspruch 1 oder 2, dadurch gekennzeichnet, daß als Katalysator eine organische Sulfonsäure, vorzugsweise p-Toluolsulfonsäure, eingesetzt wird.

4. Verfahren nach einem oder mehreren der Ansprüche 1 bis 3, dadurch gekennzeichnet, daß 2-Oxobernsteinsäureester und 2-Alkylacroleinoxim im Molverhältnis 1 : 2 bis 2 : 1 umgesetzt werden.

5. Verfahren nach einem oder mehreren der Ansprüche 1 bis 4, dadurch gekennzeichnet, daß die Umsetzung bei 50 bis 150, vorzugsweise 80 bis 100˚ C, erfolgt.

**Europäisches Patentamt**

# EUROPÄISCHER RECHERCHENBERICHT

Nummer der Anmeldung

**EP 91 11 2478**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 308 084   (IMPERIAL CHEMICAL INDUSTRIES) <br> * das ganze Dokument * <br> – – – | 1 | C 07 D 213/803 |
| A,D | EP-A-0 274 379   (SUGAI CHEMICAL INDUSTRY CO.,LTD.) <br> * Seiten 1 - 4, Zeile 22 * <br> – – – | 1 | |
| A | EP-A-0 161 221   (CIBA-GEIGY AG.) <br> * Seiten 1 - 3, Absatz 1 * <br> – – – – – | 1 | |
| | | | **RECHERCHIERTE SACHGEBIETE (Int. Cl.5)** <br><br> C 07 D 213/00 |

Der vorliegende Recherchenbericht wurde für alle Patentansprüche erstellt

| Recherchenort | Abschlußdatum der Recherche | Prüfer |
|---|---|---|
| Berlin | 25 November 91 | KYRIAKAKOU G |

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument
-------------------------------------------------------------
& : Mitglied der gleichen Patentfamilie, übereinstimmendes Dokument